# EUROPÄISCHE PATENTSCHRIFT

(11) **EP 0 626 944 B1**
(45) Veröffentlichungstag und Bekanntmachung des Hinweises auf die Patenterteilung: **06.05.1998**
(21) Anmeldenummer: 93903946.7
(22) Anmeldetag: 10.02.1993
(51) Int. Cl.: C07C 317/18, C11D 3/00

(54) **THIODIGLYKOL-DERIVATE, VERFAHREN ZU IHRER HERSTELLUNG UND DEREN VERWENDUNG ZUM WEICHMACHEN VON TEXTILIEN**
THIODIGLYCOL DERIVATIVES, PROCESS FOR PRODUCING THEM AND THEIR USE FOR SOFTENING TEXTILES
DERIVES DU THIODIGLYCOL, PROCEDE DE PREPARATION ET UTILISATION POUR L'ADOUCISSEMENT DE TEXTILES

(30) Priorität: 19.02.1992 DE 4204885
(43) Veröffentlichungstag der Anmeldung: 07.12.1994
(73) Patentinhaber: Henkel Kommanditgesellschaft auf Aktien, 40191 Düsseldorf (DE)
(72) Erfinder: DAUTE, Peter, D-4300 Essen 1 (DE); WEGENER, Ingo, D-4000 Düsseldorf 13 (DE); MEFFERT, Alfred, D-4019 Monheim (DE); BERGER, Faize, D-4000 Düsselforf 13 (DE)
(86) Internationale Anmeldenummer: EP9300323
(87) Internationale Veröffentlichungsnummer: WO9316989

(56) Entgegenhaltungen:
- DE-A- 3 936 862
- DE-A- 4 021 694
- GB-A- 1 097 396
- US-A- 3 299 145
- US-A- 3 627 845
- JOURNAL OF THE AMERICAN CHEMICAL SOCIETY. Bd. 110, Nr. 20, 28. September 1988, GASTON, PA US Seiten 6840 - 6845 J.-H. FUHRHOP ET AL. 'A Macrocyclic Tetraether Bolaamphiphile and a Oligoamino a,w-Dicarboxylate Combine To Form Monolayered, Porous Vesicle Membranes, Which Are Reversibly Sealed by EDTA and Other Bulky Anions'

## Beschreibung

Die vorliegende Erfindung betrifft die Verwendung von Thiodiglykol-Derivaten zum Weichmachen von Textilien.

Auf dem Gebiet der Textilweichspüler für Haushalt und Gewerbe kommen vor allen Dingen quartäre Ammoniumverbindungen insbesondere vom Typ Dimethyldistearylammoniumchlorid oder in jüngerer Zeit ähnlich aufgebaute Verbindungen mit einer, zwei oder drei Fettacyloxyalkylgruppen in Frage. Wegen der zunehmenden Bedeutung von Lagerstabilität und Viskositätscharakteristik sowie insbesondere der biologischen Abbaubarkeit, vor allem von hochkonzentrierten Weichspülern, sind zahlreiche Vorschläge zum Ersatz dieser Komponenten durch stickstofffreie Austauschverbindungen bzw. entsprechende Systeme veröffentlicht worden. Diese Vorschläge umfassen dabei sowohl anorganische, insbesondere anorganische unlösliche Komponenten, wie Schichtsilikatverbindungen (siehe beispielsweise die deutsche Patentschrift Nr. 23 34 899), als auch ausgewählte organische Komponenten, beispielsweise Di-Salze von langkettigen α-Sulfofettsäuren und Kombinationen solcher Systeme (siehe beispielsweise die deutsche Patentschrift Nr. 36 04 039).

Aus der deutschen Patentanmeldung DE 39 36 862 Al sind bereits Thiodiglykol-Derivate bekannt, ein Verfahren zu ihrer Herstellung und deren Verwendung zum Weichmachen von Textilien. Diese Verbindungen sind jedoch aufgrund ihrer begrenzten Wasserlöslichkeit und ihrer Schmelzpunkte nur durch aufwendiges Abmischen zu stabilen Dispersionen zu konfektionieren. Eine Verbesserung in dieser Hinsicht stellen die in der deutschen Patentanmeldung P 40 21 694.2 beschriebenen Ethoxylate von Thiodiglykol-Derivaten dar. Zu deren Herstellung geht man von α-Olefinepoxiden aus. Die Aufgabe der vorliegenden Erfindung bestand daher darin, zum Weichmachen von Textilien geeignete Thiodiglykol-Derivate unter Einsatz von nach-wachsenden Rohstoffen zu finden.

In J. Am. Chem. Soc. Vol. 110, No. 20 (1988), S. 6845 sind Bis-(2-dodecyloxyethyl)-Sulfid.Sulfoxid und -Sulfon als Synthesezwischenstufen beschrieben. Aus GB-A-1,097,396 sind ebenfalls Bis-(alkoxyethyl)-sulfone als Weichmacher für Polyvinylchlorid bekannt. In US-A-3,627,845 sind Polyoxyalkylensulfide als Tenside und antimirkobielle fungicide und insekticide Wirkstoffe beschrieben. Aus US-3,299,145 ist die Verwendung von Bis-(2-alkoxyethyl)-sulfonen mit vorzugsweise 1 bis 5 C-Atomen in der Alkoxygruppe als Textilveredelungsmittel zur Knitterfest-Ausrüstung bekannt.

Gegenstand der vorliegenden Erfindung ist daher die Verwendung von Thiodiglykol-Derivaten der allgemeinen Formel I

R¹-O-C₂H₄O)ₓ-(C₂H₄-SO_{z}-C₂H₄-O)_{w}-(C₂H₄-O)_{y}-R² (I)

mit der Bedeutung:
R¹ und R² = geradkettige oder verzweigte Alkyl- oder Alkenylgruppen mit 6 bis 30 Kohlenstoffatomen oder Wasserstoff
- x + y =: 0 bis 20
- w =: 1 bis 5
- z =: 1 oder 2,
wobei R¹ und R² gleich oder verschieden sein können, zum Weichmachen von Textilien.

Bei den erfindungsgemäß zu verwendenden Thiodiglykol-Derivaten handelt es sich also um ethoxylierte und nicht-ethoxylierte Bis-ether von Thiodigklyko Sulfoxiden bzw. -Sulfonen. Bevorzugt sind Thiodiglykol-Derivate der Formel I, deren von Fettalkoholen sich ableitende Reste R¹ und R² geradkettige Alkyl- oder Alkenylgruppen sind, die vorzugsweise 8 bis 22 Kohlenstoffatome haben. Auch wenn es grundsätzlich möglich ist, daß R¹ und R² sich von Fettalkoholen mit einer einheitlichen Anzahl von Kohlenstoffatomen ableiten, ist es aus Kostengründen zweckmäßig, natürlich vorkommende Fettalkoholgemische für die Herstellung der Thiodiglykol-Derivate zu verwenden. Beispiele für preiswert verfügbare Fettalkoholgemische sind beispielsweise Kokosölfettalkohol, Talgölfettalkohol, Palm- und Palmkernfettalkohol oder auch Erdnußölfettalkohol. Die Reste R¹ und R² können gleich oder verschieden sein; da die Herstellung von Thiodiglykol-Derivaten mit gleichen Gruppen R¹ bzw. R² aber einfacher ist, sind Thiodiglykol-Derivate, bei denen R¹ und R² gleich sind, bevorzugt. Geht man von ethoxylierten Fettalkoholen aus, erhält man dementsprechend ethoxylierte Thiodiglykol-Derivate, die ebenfalls gute Textilweichmacher darstellen. Je nachdem, inwieweit man die Oxidation der Thiodiglykol-Derivate durchführt, erhält man Sulfoxide (z = 1) oder Sulfone (z = 2). Bevorzugt sind solche Thiodiglykol-Derivate, bei denen z = 2 ist, d. h. also Thiodiglykolsulfone.

Das Verfahren zur Herstellung von Thiodiglykol-Derivaten der Formel I besteht darin, daß man Thiodiglykol mit Alkoholen der Formel R¹-OH und/oder R²-OH oder deren Ethoxylate im Molverhältnis 1 : 10 bis 10 : 1, wobei R¹ und R² die angegebene Bedeutung haben, in Gegenwart von sauren Verbindungen bei 120 bis 200 °C unter Abscheidung von Wasser unter Etherbildung kondensiert und die Kondensationsprodukte in an sich bekannter Weise zum Sulfoxid und/oder Sulfon oxidiert. In einer bevorzugten Ausführungsform des Verfahrens setzt man Thiodiglykol mit Alkoholethoxylaten der Formel R¹-O-(C₂H₄O)ₓH und/oder R²-O-(C₂H₄O)_{y}H um. Zweckmäßigerweise sind die verwendeten Alkoholethoxylate verhältnismäßig niedrig ethoxyliert, d.h., daß man Alkoholethoxylate mit höchstens 10 Mol Ethylenoxid pro Mol Alkohol verwendet. Die Umsetzungsreaktion unter Ausbildung von Ethergruppen läuft besonders glatt, wenn man als Katalysator saure Verbindungen verwendet. Bevorzugt ist daher ein Verfahren, bei dem man als Katalysator saure Verbindungen, z. B. p-Toluolsulfonsäure, Sulfobernsteinsäure oder Kaliumhydrogensulfat verwendet. Besonders hellfarbige Produkte erhält man, wenn man die Kondensationsreaktion unter Inertgasatmosphäre vornimmt. In der Regel wird man als Inertgas Stickstoff verwenden.

Im Anschluß an die Kondensationsreaktion findet die Oxidation statt. In einer bevorzugten Ausführungsform der Oxidationsreaktion nimmt man die Oxidation mit Wasserstoffperoxid vor, wobei es zweckmäßig sein kann, daß man die Oxidation in Gegenwart von organischen Säuren oder Lösungsmitteln durchführt. Bevorzugt ist die Oxidation mit Wasserstoffperoxid bei einer Temperatur im Bereich von 70 bis 100 °C.

Bevorzugt ist die Verwendung von Thiodiglykol-Derivaten der Formel I zum Weichmachen von Textilien aus natürlichen und synthetischen Fasern sowie deren Mischungen. Der Trocknungszustand des zu behandelnden Textilmaterials ist bei der erfindungsgemäßen Verwendung der Thiodiglykol-Derivate unwesentlich. Dementsprechend tritt der angestrebte Weichmachungseffekt sowohl bei der Einwirkung auf Naßgewebe wie auch bei der Einwirkung auf Trockengewebe ein. Die erfindungsgemäßen Thiodiglykol-Derivate ziehen im allgemeinen sowohl aus wäßriger Flotte als auch durch Zwangsapplikation auf das entsprechende Textil auf. Bevorzugt ist die Verwendung im Anschluß an einen Wasch- oder Veredelungsprozeß in wäßriger Flotte, die einen Gehalt an Thiodiglykol-Derivaten der Formel I im Bereich von 0,1 bis 1 g/l hat. Die wäßrigen Flotten stellt mar zweckmäßigerweise durch Verdünnen von wäßrigen Dispersionen und/oder Emulsionen, die die erfindungsgemäßen Thiodiglykol-Derivate enthalten, her. Diese Dispersionen enthalten Thiodiglykol-Derivate im Bereich von etwa 1 bis 50 Gew.-%, zweckmäßigerweise im Bereich von 5 bis 20 Gew.-%. In einer wichtigen Ausführungsform der Erfindung werden die Thiodiglykol-Derivate in Abmischung mit Hilfsstoffen eingesetzt. Als wirksame Hilfsstoffe kommen hier Tenside in breiter Definition in Betracht. Geeignete Aniontenside sind beispielsweise Fettalkoholsulfatsalze ebenso wie Alkylsulfonatsalze, wobei sich vorzugsweise die Alkali- und Erdalkalisalze und insbesondere die Natrium- und Magnesiumsalze bewährt haben. Aus der ersten Gruppen sind als besonders geeignete Mischkomponente das C₁₆-C₁₈-Alkoholsulfat und verwandte Verbindungen zu nennen. Zur zweiten Gruppe zählen beispielsweis α-Sulfofettsäuremethylestersulfonate, Di-Salze von α-Sulfofettsäuren und vergleichbare Verbindungen. Aus dem Bereich der nicht-ionischen Tenside sind sowohl die klassischen Ethoxylate als auch Alkylglycoside zu nennen. Die genannten Verbindungen können ebenso als Hilfsstoffe bei der Herstellung der Dispersionen/Emulsionen verwendet werden wie Emulgatoren anderer Verbindungsklassen beispielsweise Glyceriden, Glycerinpartialether und/oder Glycerinpartialester, die neben ein oder zwei freien Hydroxylgruppen längerkettige Kohlenwasserstoffreste in den Ether bzw. Ester bildenden funktionellen Substituenten aufweisen.

Als weitere Hilfsstoffe können Konservierungsmittel, Viskositätsstellmit-tel, saure Verbindungen, Farbstoffe und Duftstoffe verwendet werden. Der pH-Wert der Dispersionen/Emulsionen kann in einem weiten Bereich variieren, beispielsweise im Bereich von 4 bis 11, vorzugsweise im Bereich von etwa 5 bis 7.

Außer den genannten Hilfsstoffen können die zum Weichmachen von Textilien zu verwendenden Dispersionen/Emulsionen noch weitere Bestandteile enthalten. Beispielsweise Lösungsmittel oder Stabilisatoren. Es ist bevorzugt, die Thiodiglykol-Derivate der Formel I in Abmischung mit Lösungsmitteln, Dispergierhilfsmitteln, Emulgatoren und/oder Stabilisatoren in Mengen von 5 bis 95 Gew.-%, vorzugsweise von 20 bis 70 Gew.-%, bezogen auf das Wirkstoffgemisch, zu verwenden, wobei es wesentlich ist, daß die Thiodiglykol-Derivate in feiner Verteilung vorliegen, um optimale Effekte zu erzielen.

Im folgenden werden beispielhaft unterschiedliche Thiodiglykol-Derivate, deren Herstellung und deren Verwendung zum Weichmachen von Textilien beschrieben, ohne daß die Erfindung darauf beschränkt ist.

### Beispiele

### Beispiel 1

Dieses Beispiel beschreibt die Herstellung eines typischen erfindungsgemäßen Thiodiglykol-Derivats. In analoger Weise können unter Einsatz unterschiedlicher Alkohole, gegebenenfalls in unterschiedlichen Molverhältnissen mit unterschiedlichen Mengen an Wasserstoffperoxid andere erfindungsgemäße Thiodiglykol-Derivate hergestellt werden.

In ein Reaktionsgefäß mit Rühreinrichtung und Wasserabscheider wurden 3787 g (14 Mol) Stearylalkohol, 855 g (7 Mol) Thiodiglykol (Glyecin A, BASF) und 11,4 g p-Toluolsulfonsäuremonohydrat eingewogen und unter Stickstoffeinleitung und Rühren 6 Stunden auf ca. 170 °C erhitzt. Über die abgeschiedene Wassermenge wurde der Reaktionsverlauf kontrolliert. Man erhielt 4400 g Thiodiglykoldistearylether als leicht gelb gefärbten Feststoff.

Zur Oxidation zum Sulfon wurden 1270 g des wie zuvor beschrieben erhaltenen Thiodiglykoldistearylethers auf 80 °C erwärmt und innerhalb von ca. 60 Minuten langsam mit 433 g 35 %igen Wasserstoffperoxid versetzt. Anschließend wurde 4 h lang bei 90 bis 98 °C nachgerührt und danach das Reaktionsprodukt wiederholt mit heißem Wasser gewaschen, bis im Waschwasser kein Peroxid mehr nachweisbar war. Das schwach gelbe feste Produkt wurde anschließend im Vakuum bei 120 °C getrocknet. Man erhielt 1244 g des Sulfons.

### Beispiel 2

Dieses Beispiel beschreibt die Verwendung und die Prüfung der erfindungsgemäßen Thiodiglykol-Derivate.

Baumwoll-Frottierlappen wurden durch 1-maliges Waschen bei 95 °C und 5-maliges Waschen bei 60 °C in einer Lauge, die 215 g eines phosphatfreien Waschmittels pro 3,5 kg Frottierlappen ohne Vorwäsche gehärtet. Diesem gehärtetem Material wurde die Griffnote 0 zugeordnet. Je 60 g gehärtete Frottierlappen wurden in einem Wackergerät mit 600 g einer wäßrigen Dispersion, die ein Gemisch aus 5 Gew.-% des wie zuvor beschriebenen erhaltenen Sulfons und 4 Gew.-% Talgalkoholsulfat-Natrium (Flottenverhältnis von 1 : 10), in Wasser mit einer Härte von 16 °d enthielt, 5 Minuten lang behandelt. Nach dem Trocknen wurde die Weichheit der Frottierlappen durch Personen ermittelt, die in der Beurteilung der Weichheit geübt sind. Einem "sehr weichen" Griff wurde die Griffnote 4 zugeordnet. Frottierlappen, die mit einer Behandlungsflotte, die 3 g der oben beschriebenen wäßrigen Dispersion pro Liter enthielt, behandelt waren, erhielten die Griffnote 3; Frottierlappen, die mit einer Behandlungsflotte der doppelten Konzentration behandelt worden waren, erhielten die Griffnote 4.

Vergleichbare Ergebnisse erhielt man, wenn n-0ctylalkohol oder ein Gemisch aus n-Octylalkohol mit Stearylalkohol oder Behenhylalkohol anstelle von Stearylalkohol zur Etherbildung benutzt wurde. Ein ebenfalls gutes Ergebnis erhielt man, wenn ein Produkt eingesetzt wurde, bei dessen Etherbildung der Alkohol im Unterschuß bzw. im Überschuß eingesetzt wurde. Die Griffnoten der damit behandelten Prüftextilien lagen im Bereich von 3,0 bis 3,4 bei einer Anwendungskonzentration von 3 g pro Liter.

### Beispiel 3

900 Gramm einer Mischung von Cetyl- und Stearylalkohol (1:1) wurden mit 7,5 Gramm einer 30 %igen Lösung von Kaliumhydroxid in Methanol versetzt und in einem Autoklaven auf 80 °C erhitzt. Bei dieser Temperatur wurden die vorhandenen Methanolspuren durch 5-maliges Evakuieren und Belüften mit Stickstoff entfernt. Nach Erhöhen der Reaktionstemperatur auf 140 °C wurden insgesamt 154 Gramm Ethylenoxid portionsweise so zudosiert, daß der Druck im Reaktor einen Wert von 5.10⁵ Pa nicht überstieg. Nach Beendigung der Reaktion wurde auf ca. 90 °C gekühlt und zur Abtrennung noch vorhandener Ethylenoxidspuren 15 Minuten lang evakuiert. Es wurde ein farbloser Feststoff mit einer OH-Zahl von 185 erhalten.

### Ausbeute: 1061 g Fettalkohol-Ethoxylat.

Analog Beispiel 1 wurden 760 Fettalkohol-Ethoxylat, 153 Gramm Thiodiglykol und 15 Gramm p-Toluolsulfonsäure-Monohydrat umgesetzt (4,5 Stunden, 160 - 180 °C, abgeschiedene Wassermenge 46 ml).

Analog Beispiel 1 wurde mit 255 Gramm 35-prozentigem Wasserstoffperoxid oxidiert. Ausbeute: 838 Gramm, OH-Zahl = 24, Säurezahl = 1,4.

### Beispiel 4

Analog Beispiel 1 wurden 1061 Gramm 2-Hexyldecanol, 244 Gramm Thiodiglykol und 3,6 Gramm p-Toluolsulfonsäure-Monohydrat umgesetzt (5 Stunden, 160 bis 170 °C, abgeschiedene Wassermenge 66 ml).

Analog Beispiel 1 wurde mit 393 Gramm 35-prozentigem Wasserstoffperloxid oxidiert. Ausbeute: 1235 Gramm, OH-Zahl = 54, Säurezahl = 0,8.

### Beispiel 5

Analog Beispiel 1 wurde mit 1117 Gramm Oleylalkohol (Jod-Zahl = 90-95), 244 Gramm Thiodiglykol und 3,6 Gramm p-Toluolsulfonsäure-Monohydrat umgesetzt (5,5 Stunden, 160 bis 170 °C, abgeschiedene Wassermenge 65 ml).

Analog Beispiel 1 wurden 393 Gramm 35 %igem Wasserstoffperoxid oxidiert. Ausbeute: 1222 Gramm, OH-Zahl = 54, Säurezahl = 0,2, Jod-Zahl = 76.

### Beispiel 6

Analog Beispiel 1 wurden 866 Gramm eines technischen Alkoholgemisches (5% C₈, 5% C₁₀, 50% C₁₂, 20% C₁₄, 10% C₁₆, 8% C₁₈), 269 Gramm Thiodiglykol und 4,0 Gramm p-Toluolsulfonsäure-Monohydrat umgesetzt (6 Stunden, 160 bis 170 °C, abgeschiedene Wassermenge 70 ml).

Analog Beispiel 1 wurde mit 449 Gramm 35-prozentigem Wasserstoffperoxid oxidiert.
OH-Zahl = 55, Säurezahl = 0,6.

### Beispiel 7

886,9 Gramm 1-Hexadecenoxid und 315,3 Gramm Thiodiglykol + 2 EO (mit 2 Mol Ethylenoxid umgesetztes Thiodiglykol) wurden mit 7,8 Gramm einer 50 %igen Kalilauge in einem Reaktionsgefäß vorgelegt und die vorhandenen Wasserspuren unter Erhitzen auf 100 °C im Vakuum entfernt. Die leicht exotherm reagierende Mischung wurde dann unter Stickstoff auf etwa 160 bis 170 °C erwärmt. Zur Kontrolle der Reaktion wurde in regelmäßigen Abständen der Epoxid-Sauerstoff-Gehalt im Reaktionsgemisch bestimmt, wobei nach 3 bis 6 Stunden Reaktionszeit ein Epoxid-Sauerstoff-Gehalt von unter 0,3 % erreicht wurde und die Reaktion damit beendet war. Nach Abkühlen wurde das Produkt mit einer äquivalenten Menge 90 %-iger Milchsäure neutralisiert.

1100 Gramm des wie zuvor beschrieben hergestellten Umsetzungsproduktes wurden auf 80 °C erwärmt und innerhalb von 60 Minuten langsam mit 153 Gramm 70 %igem Wasserstoffoxid versetzt. Während der weiteren Reaktion wurde die Temperatur durch Kühlen auf unter 90 °C gehalten. Zur Nachreaktion wurde das Produkt noch 4,5 Stunden auf 90 °C erwärmt und danach solange mit heißem Wasser gewaschen, bis der Peroxidtest im Waschwasser negativ ausfiel. Das leicht gelbe, feste Sulfon wurde anschließend im Vakuum bei 120 °C getrocknet.
OH-Zahl = 149, Säurezahl = 0,9.

### Beispiel 8:

611 g (5 Mol) Thiodiglykol und 3,1 g p-Toluosulfonsäure-Monohydrat wurden in einem Reaktionsgefäß mit Rührapparatur und Wasserabschneider vorgelegt und unter Stickstoffanleitung auf 140 - 180 °C (3,5 h) erhitzt. Über die ausgetragene Wassermenge wurde der Kondensationsverlauf kontrolliert (abgeschiedene Wassermenge 45 ml + 30 ml Thioxan). Nach Zugabe von 1353 g (4,5 Mol) Stearylalkohol wurde weitere 3 h auf 170 °C erhitzt (Wassermenge 80 ml).

Das Reaktionsprodukt wurde analog Beispiel 1 mit 895 g 35 %igem Wasserstoffperoxid oxidiert. Ausbeute 1627 g, OHZ = 16, SZ = 0,8.

### Beispiel 9:

Analog Beispiel 1 wurden 651 g Octanol, 306 g Thiodiglykol und 4,6 g p-Toluolsulfonsäure-Monohydrat umgesetzt (6 h, 160 - 180 °C, abgeschiedene Wassermenge 80 ml) und mit 495 g 35 %igem Wasserstoffperoxid oxidiert. Ausbeute 758 g, OHZ = 36, SZ = 0,6.

## Patentansprüche

1. Verwendung von Thioglycolderivaten der Formel I
R¹-O-(C₂H₄O)ₓ-(C₂H₄-SO_{z}-C₂H₄-O)_{w}-(C₂H₄-O)_{y}-R² (I)
mit der Bedeutung:
R¹ und R² = geradkettige oder verzweigte Alkyl- oder Alkenylgruppen mit 6 bis 30 Kohlenstoffatomen oder Wasserstoff
x + y = 0 bis 20
w = 1 bis 5
z = 1 oder 2,
wobei R¹ und R² gleich oder verschieden sein können, zum Weichmachen von Textilien.

2. Verwendung nach Anspruch 1, dadurch gekennzeichnet, daß die Textilien im Anschluß an einen Wasch- oder Veredelungsprozeß mit einer wäßrigen Flotte mit einem Gehalt von 0,1 - 1 g/l an Thioglycolderivaten der Formel I behandelt werden.

3. Verwendung nach Anspruch 1 oder 2, dadurch gekennzeichnet, daß die Textilien mit einer wäßrigen Flotte behandelt werden, die durch Verdünnen einer wäßrigen Dispersion oder Emulsion, enthaltend 1 - 50 Gew.-% Thioglycolderivate der Formel I in Abmischung mit Tensiden, insbesondere Fettalkoholsulfatsalzen, hergestellt wird.

## Claims

1. The use of thioglycol derivatives corresponding to formula I:
R¹-O-(C₂H₄O)ₓ-(C₂H₄-SO_{z}-C₂H₄-O)_{w}-(C₂H₄-O)_{y}-R² (I)
in which
R¹ and R² may be the same or different and represent linear or branched alkyl or alkenyl groups containing 6 to 30 carbon atoms or hydrogen,
x + y = 0 to 20,
w = 1 to 5,
z = 1 or 2,
for softening fabrics.

2. The use claimed in claim 1, characterized in that, after a washing or finishing process, the fabrics are treated with an aqueous liquor containing 0.1 to 1 g/l of thioglycol derivatives corresponding to formula I.

3. The use claimed in claim 1 or 2, characterized in that the fabrics are treated with an aqueous liquor prepared by diluting an aqueous dispersion or emulsion containing 1 to 50% by weight of thioglycol derivatives corresponding to formula I in admixture with surfactants, more particularly fatty alcohol sulfate salts.

## Revendications

1. Utilisation de dérivés de thioglycol de la formule I
R¹-O-(C₂H₄O)ₓ-(C₂H₄-SO_{z}-C₂H₄-O)_{w}-(C₂H₄-O)_{y}-R² (I)
avec la signification :
R¹ et R² = des groupes alkyle ou alcényl à chaîne droite ou ramifiée avec 6 à 30 atomes de carbone ou de l'oxygène
x + y = 0 à 20
w = 1 à 5
z = 1 ou 2
R¹ et R² pouvant être égaux ou différents, pour adoucir les textiles.

2. Utilisation selon la revendication 1,
caractérisée en ce que
les textiles sont traités en liaison avec un processus de lavage ou de valorisation par un bain aqueux ayant une teneur de 0,1-1 g/l de dérivés de thioglycol de la formule I.

3. Utilisation selon les revendications 1 ou 2,
caractérisée en ce que
les textiles sont traités par un bain aqueux qui est produit par dilution d'une dispersion ou émulsion aqueuse, contenant de 1 à 50 % en poids de dérivés de thioglycol de la formule I en mélange avec des tensioactifs, en particulier des sels de sulfate d'alcool gras.
